# EUROPÄISCHE PATENTSCHRIFT

(11) **EP 2 464 636 B1**
(45) Veröffentlichungstag und Bekanntmachung des Hinweises auf die Patenterteilung: **08.07.2015**
(21) Anmeldenummer: 10742095.2
(22) Anmeldetag: 04.08.2010
(51) Int. Cl.: C07D 307/60

(54) **VERFAHREN ZUM HERSTELLEN VON 2,4-DIOXOTETRAHYDROFURAN-3-CARBOXYLATEN**
PROCESS FOR THE SYNTHESIS OF 2,4-DIOXOTETRAHYDROFURAN-3-CARBOXYLATES
PROCÉDÉ DE SYNTHÈSE DE 2,4-DIOXOTETRAHYDROFURAN-3-CARBOXYLATES

(30) Priorität: 11.08.2009 EP 09167593
(43) Veröffentlichungstag der Anmeldung: 20.06.2012
(73) Patentinhaber: Bayer Intellectual Property GmbH, 40789 Monheim am Rhein (DE)
(72) Erfinder: LUI, Norbert, 51519 Odenthal (DE); HEINRICH, Jens-Dietmar, 51399 Burscheid (DE); FUNKE, Christian, 42799 Leichlingen (DE)
(74) Vertreter: BIP Patents
(86) Internationale Anmeldenummer: PCT/EP2010/004777
(87) Internationale Veröffentlichungsnummer: WO 2011/018180

(56) Entgegenhaltungen:
- ERICH BENARY: BERICHTE DER DEUTSCHEN CHEMISCHEN GESELLSCHAFT, VERLAG CHEMIE. WEINHEIM, DE, Bd. 44, 1. Januar 1911 (1911-01-01), Seiten 1759-1765, XP009097711 in der Anmeldung erwähnt
- MULHOLLAND T P C ET AL: "A synthesis of tetronic acid [furan-2(3H),4(5H)-dione] and three analogues" JOURNAL OF THE CHEMICAL SOCIETY. PERKIN TRANSACTIONS 1, CHEMICAL SOCIETY. LETCHWORTH, GB, Nr. 9-10, 1. Januar 1972 (1972-01-01), Seiten 1225-1231, XP009122071 ISSN: 0300-922X
- REDDY S H K ET AL: "Anodic oxidations of electron-rich olefins: radical cation based approaches to the synthesis of bridged bicyclic ring skeletons", TETRAHEDRON, ELSEVIER SCIENCE PUBLISHERS, AMSTERDAM, NL, vol. 57, no. 24, 11 June 2001 (2001-06-11) , pages 5183-5197, XP004243444, ISSN: 0040-4020, DOI: 10.1016/S0040-4020(01)00358-1
- Kurt Schank ET AL: "Eine einstufige Synthese des Decalin-dion-(1.8)-Systems", Chemische Berichte, vol. 102, no. 1, 1 January 1969 (1969-01-01), pages 71-76, XP055140744, ISSN: 0009-2940, DOI: 10.1002/cber.19691020110

## Beschreibung

Die vorliegende Erfindung betrifft ein Verfahren zur Herstellung von 2,4-Dioxotetrahydrofuran-3-carboxylaten.

2,4-Dioxotetrahydrofuran-3-carboxylate sind wichtige Zwischenverbindungen bei der Wirkstoffherstellung. Sie können nach verschiedenen bekannten Methoden hergestellt werden.

Beispielsweise beschreiben Athanasellis et al. (Synlett, 2002 (10), 1736 - 1738) eine durch Hydroxybenzotriazol vermittelte mehrstufige Synthese von 3-Methoxycarbonyltetronsäuren, d.h. 2,4-Dioxotetrahydrofuran-3-carboxylaten, unter Verwendung von O-geschützten α-Hydroxysäuren und einem Malonsäureester unter anschließender Cyclisierung in Gegenwart von Natriumhydrid. Durch die Reaktion entsteht elementarer Wasserstoff.

Mitsos et al. (Journal of Heterocyclic Chemistry, 39 (6), 1201 - 1205) beschreiben ebenfalls die Herstellung von 2,4-Dioxotetrahydrofuran-3-carboxylaten. Durch eine durch N,N'-Dicyclohexylcarbodiimid vermittelte Kondensationsreaktion von O-geschützten α-Hydroxysäuren mit N-Hydroxysuccinimide wird zuerst der N-Succinimidylester einer α-Acetoxysäure hergestellt, der dann mit einem Malonsäuredialkylesteranion, das durch Reaktion von Malonsäureester und Natriumhydrid in wasserfreiem Benzol erzeugt wurde, reagiert. Durch die Reaktion entsteht elementarer Wasserstoff. Die Reaktionsmischung wird wässrig aufgearbeitet und durch Ansäuern das gewünschte Produkt erhalten.

Die von Athanasellis und Mitsos beschriebenen Verfahren haben den Nachteil, dass sie nicht kosteneffizient großtechnisch eingesetzt werden können. Die verwendeten Chemikalien sind teuer und die Verfahren sind komplex, weil sie viele Verfahrensschritte beinhalten und gleichzeitig Natriumhydrid einsetzen. Der Umgang mit Natriumhydrid ist generell nicht wünschenswert, denn es ist leicht entzündlich und man muss unter Ausschluss von Wasser arbeiten. Auch muss der bei der Reaktion entstehende elementare Wasserstoff aus dem Reaktionssystem entfernt werden, was weitere Verfahrensschritte und besondere Sicherheitsmaßnahmen bedingt.

Ein Verfahren zur Herstellung von 3-Ethoxycarbonyl-4-hydroxyfuran-2(5H)-on, die Enolform von 2,4-Dioxotetrahydrofuran-3-ethoxylat, das auf die Verwendung von Natriumhydrid verzichtet ist bei Campbell et al. (J. Chem. Soc. Perkin Trans I 1985, 1567 - 1576) beschrieben (Schema 1).

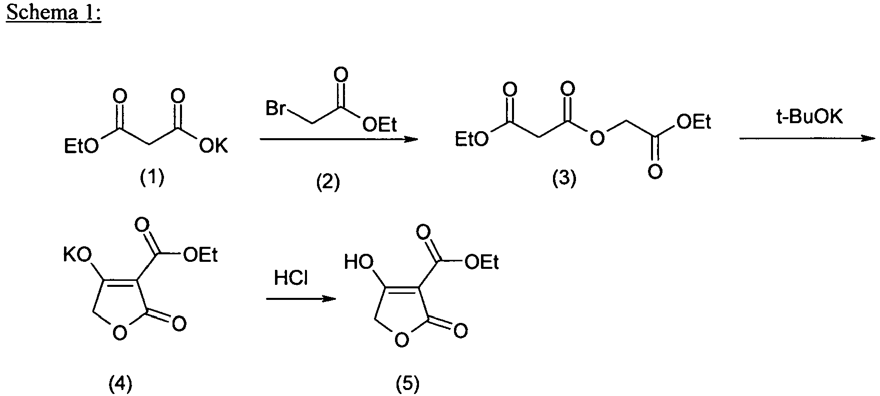

Hier wird jedoch vom Malonsäureethylestermono-Kaliumsalz (1) ausgegangen, das in einem ersten Schritt mit Bromessigsäureethylester (2) in kochenden Ethanol zu Ethoxycarbonylmethylethylmalonat (3) umgesetzt wird, das dann weiterhin mit Kalium-tert-butylat in tert-Butanol zum Kaliumsalz des 3-Ethoxycarbonyl-4-hydroxyfuran-2(5H)-on (4) weiterreagiert. Durch Zugabe von Salzsäure in Ethanol wird 3-Ethoxycarbonyl-4-hydroxyfuran-2(5H)-on (5) erhalten. Das Verfahren ist ebenfalls zur kosteneffzienten großtechnischen Anwendung ungeeignet, da auch hier die verwendeten Chemikalien teuer sind und das Verfahren viele Verfahrensschritte vorsieht.

Gleiches gilt für das in WO 2009/036899 beschriebene Verfahren zur Herstellung von 2,4-Dioxotetrahydrofuran-3-carboxylaten. Hier wird auch in einem mehrstufigen Prozess vom Malonsäureethylestermono-Kaliumsalz ausgegangen und durch Acylierungsreaktion und anschließendem Ringschluss das Salz des 3-Alkoxycarbonyl-4-hydroxyfuran-2(5H)-on erhalten. Die eingesetzten Chemikalien sind zwar weniger teuer als die im Verfahren von Campbell et al. verwendeten, die Anzahl der Verfahrensschritte ist jedoch gleich hoch, was die großtechnische Anwendung erschwert bzw. unrentabel macht.

Ein Verfahren zur Herstellung von Ethyl-2,4-dioxotetrahydrofuran-3-carboxylat, das mit weniger Verfahrensschritten als die vorbenannten Verfahren auskommt, jedoch großtechnisch nicht einsetzbar ist, wurde von Benary beschrieben (Benary, Berichte der Deutschen Chemischen Gesellschaft (1911), 44, 1759 - 1765). In diesem Verfahren wird der eingesetzte Malonsäuredimethylester zusammen mit Natrium in absolutem, d.h. wasserfreiem, Diethylether unter Freisetzung von elementaren Wasserstoff zur entsprechenden Natriumverbindung in einer Redoxreaktion umgesetzt. Bei Eiskühlung wird dann Chloracetylchlorid, das in Diethylether gelöst ist, zugegeben. Die Reaktionsmischung wird dann 24 Stunden stehen gelassen. Neben dem Freiwerden von elementaren Wasserstoff ist auch der Einsatz von Natrium in wasserfreien Lösungsmitteln für eine großtechnische Anwendung nicht vorteilhaft, weil zu aufwendig und teuer.

Ausgehend von den bekannten Verfahren zur Herstellung von 2,4-Dioxotetrahydrofuran-3-carboxylaten bzw. den entsprechenden Enol-Tautomeren stellt sich nun die Aufgabe, wie diese einfach und kostengünstig hergestellt werden können, so dass das Verfahren auch zur großtechnischen Herstellung von 2,4-Dioxotetrahydrofuran-3-carboxylaten verwendet werden kann. Unter kostengünstigen Verfahren werden solche Verfahren verstanden, die ohne großen finanziellen Aufwand durchzuführen sind, weil die Ausgangsstoffe beispielsweise kostengünstig und/oder ungefährlich sind, das Verfahren mit wenigen Verfahrensschritten auskommt bzw. sogar als "Eintopfreaktion" durchzuführen ist, und/oder das gewünschte 2,4-Dioxotetrahydrofuran-3-carboxylat in einer ausreichend hohen Ausbeute und Reinheit erhalten wird.

Es wurde nun ein Verfahren zur Herstellung von 2,4-Dioxotetrahydrofuran-3-carboxylaten gefunden, das die vorgenannten Nachteile vermeidet und das einfach und kostengünstig durchführbar ist, insbesondere weil das gefundene Verfahren in einer Eintopfreaktion durchgeführt werden kann und weil es ohne teure und/oder gefährliche Chemikalien auskommt.

Gegenstand der Erfindung ist somit das unten beschriebene Verfahren zur Herstellung von 2,4-Dioxotetrahydrofuran-3-carboxylaten der Formel (I) 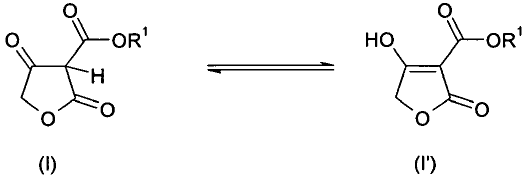 worin R¹ für die weiter unten definierten chemischen Gruppierungen steht.

Verbindungen der Formel (I) können aufgrund der Keto-Enol-Tautomerie in unterschiedlichen tautomeren Formen vorliegen. Im Rahmen der vorliegenden Erfindung sind - unabhängig von der Darstellungsweise der Verbindung der allgemeinen Formel (I) - alle tautomere Strukturen der allgemeinen Formel (I), insbesondere die der Formel (I') umfasst.

Das erfindungsgemäße Verfahren zur Herstellung von 2,4-Dioxotetrahydrofuran-3-carboxylaten der Formel (I) umfasst die folgende Schritte
(i) Umsetzen einer Halogenacetylchloridverbindung der Formel (II) 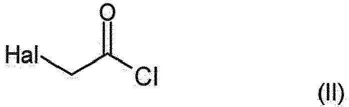 in welcher Hal für Brom, Chlor oder Iod, bevorzugt für Brom oder Chlor, besonders bevorzugt für Chlor steht,
   mit einem Malonsäureester der Formel (III) 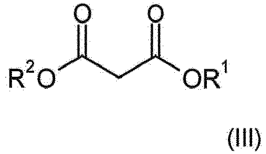 worin
   - R¹ und R²: für C₁₋₁₂-Alkyl, C₁₋₁₂-Halogenalkyl, C₅₋₁₈-Aryl, C₅₋₁₈-Halogenaryl, C₇₋₁₉-Alkylaryl, C₇₋₁₉-Arylalkyl stehen, wobei R¹ und R² für die gleichen chemischen Gruppierungen stehen, oder für ein Alkoxyalkyl der Formel -[A-O]ₘ-B stehen, worin A für C₂₋₄-Alkandiyl (Alkylen), B für C₁₋₆-Alkyl und m für 1 oder 2 steht; bevorzugt stehen R¹ und R² für Methyl, Ethyl, Isopropyl, Propyl, Benzyl oder 2-Methoxyethyl, besonders bevorzugt für Methyl oder Ethyl,
   in Gegenwart von Natriummethylat (als Base) und gegebenenfalls in Gegenwart eines Lösungsmittels;
(ii) Zugeben einer ausreichenden Menge an Wasser zum Reaktionsgemisch; und
(iii) Abtrennen des gewünschten 2,4-Dioxotetrahydrofuran-3-carboxylats.

Geeignete Basen sind alle Basen, die den Malonsäureester der Formel (III) zu deprotonieren vermögen. Besonders gut geeignete Basen sind Alkoholatbasen und haben die allgemeine Formel X(OR³)_{y}, worin X für ein Alkalimetallkation (z.B. Na⁺ oder K⁺) oder für ein Erdalkalimetallkation (z.B. Mg²⁺) steht, und R³ für C₁₋₁₂-Alkyl, bevorzugt Methyl und Ethyl steht, y steht für 1, wenn X für eine Alkalimetallkation steht, und y steht für 2 wenn X für ein Erdalkalimetallkation steht. Geeignete Basen sind beispielsweise Kalium-tert-butylat und Natriummethylat, wobei aus ökonomischen Gründen und erfindungsgemäß Natriummethylat eingesetzt wird.

Unter ausreichender Menge an Wasser wird die Menge an Wasser verstanden, die ausreicht, um das gewünschte 2,4-Dioxotetrahydrofuran-3-carboxylat der Formel (I) zu erhalten und um diese Verbindung vom Reaktionsgemisch abzutrennen. Vorzugsweise liegt das Verhältnis von Halogenacetylchlorid der Formel (II) zu Wasser im Bereich von etwa 1: 0,5 bis etwa 1: 100, insbesondere von etwa 1: 0,8 bis etwa 1: 50, speziell von etwa 1:1 bis etwa 1:30. Größere Verhältnisse sind möglich, jedoch wirtschaftlich nicht sinnvoll.

Im Schritt (i) ist es erfindungsgemäß bevorzugt, den Malonsäureester der Formel (III) entweder ohne Lösungsmittel, d.h. in Substanz, oder in einem geeigneten Lösungsmittel zu erhitzen. Danach wird die ggf. in einem geeigneten Solvens gelöste Base (z.B. Natriummethylat in Methanol) zugegeben, wobei vorzugsweise die entstehende Verbindung H_{y}(OR³)_{y} und/oder das Solvens (z.B. Methanol) gleichzeitig abdestilliert wird. Die Zugabe der Base erfolgt gewöhnlicherweise bei hohen Temperaturen, vorzugsweise bei einer Innentemperatur im Bereich von etwa 50°C bis etwa 250°C, insbesondere im Bereich von etwa 80°C bis etwa 150°C. Innentemperaturen im Bereich von etwa 90°C bis etwa 150°C sind besonders bevorzugt. Das Zugeben und/oder die Reaktion der Verbindung der Formel (II) mit dem entstandenen Malonsäureestersalz erfolgt vorzugsweise bei Innentemperaturen im Bereich von etwa 5°C bis etwa 35°C, insbesondere im Bereich von etwa 10°C bis etwa 25°C.

Das Abtrennen des gewünschten 2,4-Dioxotetrahydrofuran-3-carboxylats der allgemeinen Formel (I) aus dem Reaktionsgemisch kann beliebig erfolgen. Bevorzugt sind Filtration und/oder Phasentrennung.

2-Halogenacetylchloride der Formel (II) und Malonsäureester der Formel (III) sind käuflich erhältlich oder können nach bekannten Verfahren hergestellt werden.

Bevorzugte Malonsäureester der Formel (HI) sind solche in denen R¹ und/ oder R² für Methyl, Ethyl, Isopropyl, Propyl, Butyl, Allyl, Benzyl, Alkoxyalkyl oder C₁₋₁₂-Halogenalkyl steht, insbesondere für Methyl, Ethyl, n-Propyl oder Butyl steht.

Das erfindungsgemäße Verfahren kann anhand des folgenden Schemas verdeutlicht werden. 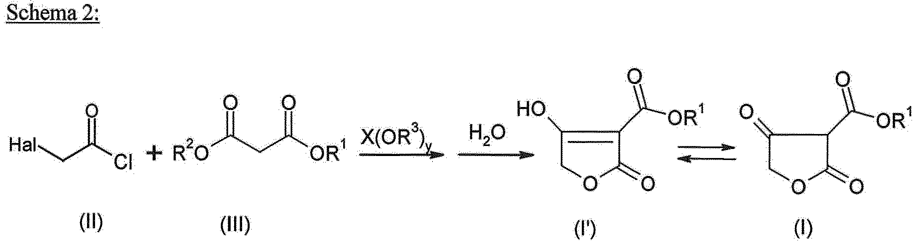 worin Hal, R¹, R², X, R³ und y die vorgenannten Bedeutungen haben und als Base X(OR³)_{y} Natriummethylat eingesetzt wird.

Die Umsetzung des Malonsäureesters der Formel (III) mit Base und anschließend mit 2-Halogenacetylchlorid der Formel (II) kann in Gegenwart eines Lösungsmittels oder in Substanz erfolgen. Vorzugsweise wird die Reaktion in einem Lösungsmittel durchgeführt. Die Lösungsmittel werden vorzugsweise in einer solchen Menge eingesetzt, dass das Reaktionsgemisch während des ganzen Verfahrens gut rührbar bleibt. Als Lösungsmittel zur Durchführung des erfindungsgemäßen Verfahrens kommen alle unter den Reaktionsbedingungen inerten organischen Lösungsmittel in Frage. Unter Lösungsmittel werden erfindungsgemäß auch Gemische reiner Lösungsmittel verstanden.

Erfindungsgemäß geeignete Lösungsmittel sind insbesondere Ether (z.B. Ethylpropylether, Methyl-tert-butylether, *n*-Butylether, Anisol, Phenetol, Cyclohexylmethylether, Dimethylether, Diethylether, Dimethylglycol, Diphenylether, Dipropylether, Diisopropylether, Di-*n*-butylether, Diisobutylether, Diisoamylether, Ethylenglycoldimethylether, Isopropylethylether, Diethylenglykoldimethylether, Triethylenglykoldimethylether, Tetrahydrofuran, Dioxan, und Polyether des Ethylenoxids und/oder Propylenoxids); Verbindungen wie Tetrahydrothiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid; Sulfone wie Dimethyl-, Diethyl-, Dipropyl-, Dibutyl-, Diphenyl-, Dihexyl-, Methylethyl-, Ethylpropyl-, Ethylisobutyl- und Pentamethylensulfon; aliphatische, cycloaliphatische oder aromatische Kohlenwasserstoffe (z.B. Pentan, Hexan, Heptan, Oktan, Nonan wie die sogenannten "White Spirits" mit Komponenten mit Siedepunkten im Bereich beispielsweise von 40°C bis 250°C, Cymol, Benzinfraktionen innerhalb eines Siedeintervalls von 70°C bis 190°C, Cyclohexan, Methylcyclohexan, Petrolether, Ligroin, Octan, Benzol, Toluol, Chlorbenzol, Brombenzol, Xylol); Ester (z.B. Methyl-, Ethyl-, Butyl-, Isobutylacetat, Dimethyl-, Dibutyl- oder Ethylencarbonat, Propylencarbonat); Amide (z.B. Hexamethylenphosphorsäuretriamid, Formamid, N,N-Dimethyl-acetamid, N-Methyl-formamid, *N,N*-Dimethyl-formamid, *N*,*N*-Dipropyl-formamid, *N*,*N*-Dibutyl-formainid, *N*-Methyl-pyrrolidin, *N*-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, *N-*Formyl-piperidin, N,N'-1,4-Diformyl-piperazin) oder Gemische davon.

Für die erfindungsgemäße Umsetzung werden als Lösungsmittel bevorzugt aromatische und/oder aliphatische Kohlenwasserstoffe, insbesondere Xylol, Diisopropylbenzol und Dichlorbenzol verwendet.

Als Solvens können erfindungsgemäße Lösungsmittel verwendet werden, die sich bei den Reaktionsbedingungen des Schritts (i) aus dem Reaktionsgemisch destillativ entfernen lassen, bevorzugt sind Alkohole wie beispielsweise Methanol, Ethanol, Isopropanol, Butanol (d.h. n-Butanol, tert-Butanol, 2-Butanol) und 2-(2-Ethoxyethoxy)-ethanol. Die Wahl des Solvens ist abhängig von der verwendeten Base. Wird eine Alkoholatbase verwendet, dann wird bevorzugt der entsprechende Alkohol als Solvens eingesetzt.

Das erfindungsgemäßen Verfahren kann im Allgemeinen im Vakuum, bei Normaldruck oder unter Überdruck durchgeführt werden.

Die angewendeten Temperaturen können in Abhängigkeit der Ausgangsstoffe variieren. Das erfindungsgemäße Verfahren kann bei Temperaturen im Bereich von etwa 0°C bis etwa 250°C, vorzugsweise bei Innentemperaturen im Bereich von etwa 10°C bis etwa 180°C durchgeführt werden. Bevorzugt wird das Verfahren bei Normaldruck und bei Innentemperaturen im Bereich von etwa 20°C bis etwa 150°C durchgeführt. Die Reaktion mit der Base kann bei höheren Innentemperaturen, insbesondere im Bereich von etwa 50°C bis 250°C, und die Umsetzung mit Halogenacetylchlorid der Formel (II) bei vergleichsweise niedrigen Innentemperaturen erfolgen, insbesondere bei Innentemperaturen im Bereich von etwa 0°C bis etwa 50°C.

Das Verhältnis des eingesetzten Malonsäureesters der Formel (III) zu der verwendeten Base kann variieren. Ein deutlicher Überschuss an Base ist jedoch zu vermeiden, da ein solcher Überschuss die Ausbeute der Reaktion reduziert, da die Base mit dem Halogenacetylchlorid der Formel (II) reagiert. Vorzugsweise liegt das Verhältnis von Malonsäureester der Formel (III) zur verwendeten Base im Bereich von etwa 1 : 0,8 bis etwa 1: 1,5, insbesondere von etwa 1: 0,9 bis etwa 1: 1,2, speziell von etwa 1:1 bis etwa 1: 1,1.

Das Verhältnis des eingesetzten Halogenacetylchlorids der Formel (II) zum verwendeten Malonsäureester der Formel (III) kann variieren. Ein deutlicher Überschuss ist für die Reaktion unkritisch, jedoch unwirtschaftlich. Vorzugsweise liegt das Verhältnis des eingesetzten Halogenacetylchlorids der Formel (II) zum verwendeten Malonsäureester der Formel (III) im Bereich von etwa 1:1,8 bis etwa 1: 2,5, insbesondere im Bereich von etwa 1: 1,9 bis etwa 1: 2,2, speziell im Bereich von etwa 1:2 bis etwa 1:2,1.

Die Bezeichnung "Alkyl" in Alleinstellung oder in Kombination mit anderen Begriffen, wie beispielsweise Arylalkyl bezieht sich auf lineare oder verzweigte gesättige Kohlenwasserstoffketten mit bis zu 12 Kohlenstoffatomen, d.h. C₁₋₁₂-alkyl, bevorzugt mit bis zu 6 Kohlenstoffen, d.h. C₁₋₆-Alkyl, besonders bevorzugt mit bis zu 4 Kohlenstoffen, d.h. C₁₋₄-Alkyl. Beispiele solcher Alkyle sind Methyl, Ethyl, n- oder iso-Propyl, n-, iso-, sec- oder tert-Butyl, n-Pentyl, n-Hexyl, n-Heptyl, n-Octyl, n-Nonyl, n-Decyl, n-Undecyl und n-Dodecyl. Die Alkyle können mit einem geeignetem Substituenten substituiert sein.

Die Bezeichnung "Aryl" - in Alleinstellung oder in Kombination mit anderen Begriffen - bezieht sich auf cyclische aromatische nicht-kondensierte oder kondensierte Gruppen, die 5 bis 18 Kohlenstoffatome aufweisen. Bevorzugte Aryle weisen 6 bis 14 Kohlenstoffatome auf (z.B. Phenyl oder Naphtyl). Unter den Arylen ist Phenyl besonders bevorzugt.

Die Bezeichnung "Arylalkyle" steht für eine Kombination aus erfindungsgemäß definierten Resten "Aryl" und "Alkyl", wobei Arylalkyle im Allgemeinen über die Alkylgruppe gebunden werden. Beispiele hierfür sind Benzyl, Phenylethyl oder α-Methylbenzyl. Unter den Arylalkylen ist Benzyl bevorzugt.

Die Bezeichnung "Alkylaryle" steht gleichfalls für eine Kombination aus erfindungsgemäß definierten Resten "Aryl" und "Alkyl", wobei Alkylaryle im Allgemeinen über die Arylgruppe gebunden werden, wie beispielsweise Tolyl.

Die Bezeichnung "Alkandiyl" oder "Alkylen" bezieht sich auf Alkyle wie oben definiert, die jedoch über eine weitere freie Bindungsvalenz verfügen, d.h. sie verfügen über 2 Bindungstellen. Beispiele solcher Alkandiyle sind Methylen, Ethylen, Propylen und Cyclopropylen.

"Halogen" oder "Hal" steht für Fluor, Chlor, Brom oder Iod, vorzugsweise für Chlor oder Brom.

Die vorliegende Erfindung wird anhand der nachfolgenden Beispiele näher erläutert, ohne die Erfindung dabei auf diese einzuschränken.

### Herstellunsgsbeispiele:

### Beispiel 1

200 g Malonsäuredimethylester (1,5 mol) in 0,98 kg Xylol werden auf 90°C erwärmt. Innerhalb von einer Stunde werden 272 g (1,5 mol) Natriummethylat in Methanol zudosiert. Dabei wird das freigesetzte Methanol abdestilliert. Die Manteltemperatur wird bis auf 110°C erwärmt (Innentemperatur 100°C). Nach Abkühlen auf 25°C wird 284 g Chloressigsäurechlorid (30-%ig) in Xylol unter Gegenkühlung (Innentemperatur 25°C) zugetropft. Nach 3 Stunden Nachrühren bei 25°C werden 567 g Wasser zugetropft. Anschließend wird weitere 3 Stunden bei Raumtemperatur, d.h. etwa 20°C, nachgerührt. Dann wird die organische Phase abgetrennt und die wässrige Phase wird zur Trockene eingeengt. Man erhält 177g Methyl 2,4-dioxotetrahydrofuran-3-carboxylat was 74,8 % Ausbeute entspricht (50,6 %ig, 44,1 % Kochsalz,).
¹H-NMR (D₂O, 298K) δ: 3,73 (s, 3H), 4,42 (s, 2H)

### Beispiel 2

143 g Malonsäuredimethylester (1,05 mol) in 0,77 kg Xylol werden auf 100°C erwärmt. Innerhalb von zwei Stunden werden 180 g (1,00 mol) Natriummethylat in Methanol zudosiert. Dabei wird das freigesetzte Methanol abdestilliert. Die Manteltemperatur wird bis auf 140°C erwärmt (Innentemperatur 125°C). Nach Abkühlen auf 15°C wird 57,6 g Chloressigsäurechlorid unter Gegenkühlung (Innentemperatur 15°C) zugetropft. Nach 3 Stunden Nachrühren bei 25°C werden 18 g Wasser zugetropft. Anschließend wird 4 Stunden bei 40°C nachgerührt, filtriert und der Rückstand im Vakuum bei 40°C getrocknet. Man erhält 104 g Methyl-2,4-dioxotetrahydrofuran-3-carboxylat was 74,2 % Ausbeute entspricht (56,9 %ig als Mischung mit NaCl).

### Beispiel 3

50 g Malonsäurediethylester (0,31 mol) in 300 ml Xylol werden auf 110°C erwärmt. Innerhalb von 1 h werden 56 g (0,3 mol) Natriummethylat in Methanol zudosiert. Dabei wird das freigesetzte Methanol abdestilliert. Die Manteltemperatur wird bis auf 140°C erwärmt (Innentemperatur 125°C). Nach Abkühlen auf 15°C wird 17,6 g Chloressigsäurechlorid unter Gegenkühlung (Innentemperatur 15°C) zugetropft. Nach 3 Stunden Nachrühren bei 25°C werden 100 g Wasser zugetropft. Anschließend wird 4 h bei 40°C nachgerührt, filtriert und der Rückstand im Vakuum bei 40°C getrocknet. Man erhält 42,6 g als Methyl 2,4-dioxotetrahydrofuran-3-carboxylat und Ethyl 2,4-dioxotetrahydrofuran-3-carboxylat Mischung was 73 % Ausbeute entspricht (46 %ig als Mischung mit NaCl).
¹H-NMR (D₂O, 298K) für den Ethylester δ: 1,28 (t, 3H), 3,73 (s, 3H), 4,21 (q, 2H), 4,42 (s, 2H)

## Patentansprüche

1. Verfahren zur Herstellung von 2,4-Dioxotetrahydrofuran-3-carboxylaten der Formel (I) 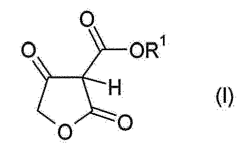 das die folgenden Schritte umfasst
(i) Umsetzen einer Halogenacetylchloridverbindung der Formel (II) 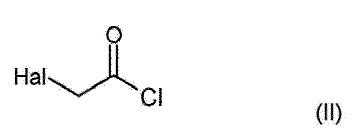 in welcher Hal für Brom, Chlor oder Iod steht;
mit einem Malonsäureester der Formel (III) 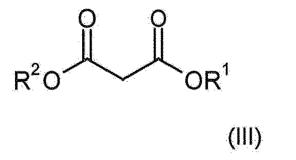 in welcher
R¹ und R² für C₁₋₁₂-Alkyl, C₁₋₁₂-Halogenalkyl, C₅₋₁₈-Aryl, C₅₋₁₈-Halogenaryl, C₇₋₁₉-Alkylaryl, C₇₋₁₉-Arylalkyl stehen, wobei R¹ und R² für die gleichen chemischen Gruppierungen stehen
oder für ein Alkoxyalkyl der Formel -[A-O]ₘ-B stehen, worin
A für C₂₋₄-Alkandiyl,
B für C₁₋₆-Alkyl und
m für 1oder 2 steht;
in Gegenwart von Natriummethylat und gegebenenfalls in Gegenwart eines Lösungsmittels;
(ii) Zugeben einer ausreichenden Menge an Wasser zum Reaktionsgemisch; und
(iii) Abtrennen des gewünschten 2,4-Dioxotetrahydrofuran-3-carboxylats.

2. Verfahren nach Anspruch 1, wobei R¹ und R² jeweils unabhängig voneinander für Methyl, Ethyl, Isopropyl, Propyl und Benzyl stehen.

3. Verfahren nach einem der Ansprüche 1 bis 2, wobei das Verhältnis von Malonsäureester der Formel (III) zur verwendeten Base im Bereich von etwa 1 : 0,8 bis etwa 1: 1,5 liegt

4. Verfahren nach einem der Ansprüche 1 bis 3, wobei das Verhältnis zwischen Halogenacetylchlorid und Wasser im Bereich von etwa 1:0,5 bis etwa 1:100 liegt.

5. Verfahren nach einem der Ansprüche 1 bis 4, wobei in Schritt (i) die Base bei einer Innentemperatur im Bereich von etwa 50°C bis etwa 250°C zur Verbindung (III) zugegeben wird.

6. Verfahren nach einem der Ansprüche 1 bis 5, wobei in Schritt (i) die Verbindung (II) bei einer Innentemperatur im Bereich von etwa 0°C bis etwa 50°C zugegeben wird.

## Claims

1. Process for preparing 2,4-dioxotetrahydrofuran-3-carboxylates of the formula (I) 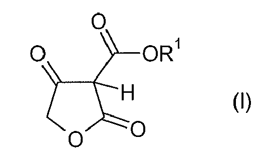 which comprises the following steps:
(i) reacting a haloacetyl chloride compound of the formula (II) 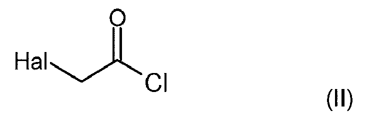 in which Hal is bromine, chlorine or iodine,
with a malonic ester of the formula (III) 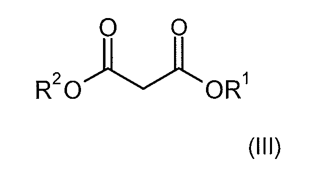 in which
R¹ and R² are C₁₋₁₂ alkyl, C₁₋₁₂ haloalkyl, C₅₋₁₈ aryl, C₅₋₁₈ haloaryl, C₇₋₁₉ alkylaryl or C₇₋₁₉ arylalkyl, wherein R¹ and R² are the same chemical moieties,
or are an alkoxyalkyl of the formula -[A-O]ₘ-B, in which
A is C₂₋₄ alkanediyl,
B is C₁₋₆ alkyl and
m is 1 or 2;
in the presence of sodium methoxide and optionally in the presence of a solvent;
(ii) adding a sufficient amount of water to the reaction mixture; and
(iii) isolating the desired 2,4-dioxotetrahydrofuran-3-carboxylate.

2. Process according to Claim 1, wherein R¹ and R² each independently of one another are methyl, ethyl, isopropyl, propyl and benzyl.

3. Process according to any of Claims 1 to 2, wherein the ratio of malonic ester of the formula (III) to the base used is in the range from about 1:0.8 to about 1:1.5.

4. Process according to any of Claims 1 to 3, wherein the ratio between haloacetyl chloride and water is in the range from about 1:0.5 to about 1:100.

5. Process according to any of Claims 1 to 4, wherein the base is added to the compound (III) in step (i) at an internal temperature in the range from about 50°C to about 250°C.

6. Process according to any of Claims 1 to 5, wherein the compound (II) is added in step (i) at an internal temperature in the range from about 0°C to about 50°C.

## Revendications

1. Procédé pour la préparation de 2,4-dioxotétrahydrofurane-3-carboxylates de formule (I) 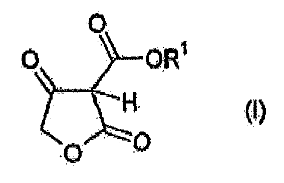 qui comprend les étapes suivantes
(i) mise en réaction d'un composé de type chlorure d'halogénoacétyle de formule (II) 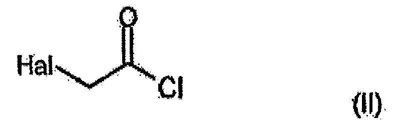 dans laquelle Hal représente le brome, le chlore ou l'iode ;
avec un ester d'acide malonique de formule (III) 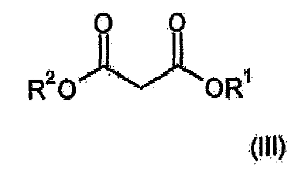 dans laquelle
R¹ et R² représentent un groupe alkyle en C₁-C₁₂, halogénoalkyle(C₁-C₁₂), aryle en C₅-C₁₈, halogénoaryle(C₅-C₁₈), alkylaryle(C₇-C₁₉), arylalkyle(C₇-C₁₉), R¹ et R² représentant les mêmes groupements chimiques
ou représentent un groupe alcoxyalkyle de formule -[A-O]ₘ-B, dans laquelle
A représente un groupe alcane (C₂-C₄)diyle,
B représente un groupe alkyle en C₁-C₆ et
m représente 1 ou 2 ;
en présence de méthylate de sodium et éventuellement en présence d'un solvant ;
(ii) addition d'une quantité suffisante d'eau au mélange réactionnel ; et
(iii) séparation du 2,4-dioxotétrahydrofurane-3-carboxylate recherché.

2. Procédé selon la revendication 1, dans lequel R¹ et R² représentent chacun indépendamment l'un de l'autre le groupe méthyle, éthyle, isopropyle, propyle ou benzyle.

3. Procédé selon l'une quelconque des revendications 1 et 2, dans lequel le rapport de l'ester d'acide malonique de formule (III) à la base utilisée se situe dans la plage d'environ 1 : 0,8 à environ 1 : 1,5.

4. Procédé selon l'une quelconque des revendications 1 à 3, dans lequel le rapport entre le chlorure d'halogénoacétyle et l'eau se situe dans la plage d'environ 1 : 0,5 à environ 1 : 100.

5. Procédé selon l'une quelconque des revendications 1 à 4, dans lequel on ajoute dans l'étape (i) la base au composé (III) à une température interne dans la plage d'environ 50 °C à environ 250 °C.

6. Procédé selon l'une quelconque des revendications 1 à 5, dans lequel on ajoute dans l'étape (i) le composé (II) à une température interne dans la plage d'environ 0 °C à environ 50 °C.
